(19) Europäisches Patentamt
European Patent Office
Office européen des brevets

(11) Numéro de publication: **0 316 213 A2**

# (12) DEMANDE DE BREVET EUROPEEN

(21) Numéro de dépôt: **88402739.2**

(22) Date de dépôt: **02.11.88**

(51) Int. Cl.⁴: **C 07 F 5/00**

(30) Priorité: **12.11.87 US 120054**

(43) Date de publication de la demande:
**17.05.89 Bulletin 89/20**

(84) Etats contractants désignés:
**AT BE CH DE FR GB IT LI NL SE**

(71) Demandeur: **RHONE-POULENC CHIMIE**
**25, quai Paul Doumer**
**F-92408 Courbevoie Cédex (FR)**

(72) Inventeur: **Gradeff, Peter S.**
**Black River Road**
**Pottersville New Jersey 07979 (US)**

**Schreiber, Fred G.**
**100 Lawrence Avenue**
**Highland Park New Jersey 08904 (US)**

**Mauermann, Heiko**
**38, Hartwell Street**
**New Brunswick New Jersey 08901 (US)**

**Grosbois, Jean**
**357 Parc de Cassan**
**F-95290 L'Isle Adam (FR)**

(74) Mandataire: **Dutruc-Rosset, Marie-Claude et al**
**RHONE-POULENC INTERSERVICES Service Brevets**
**Chimie 25, Quai Paul Doumer**
**F-92408 Courbevoie Cédex (FR)**

## (54) Hydrocarbyloxy-nitrates cériques et leur procédé de fabrication.

(57) L'invention a pour objet des hydrocarbyloxy-nitrates cériques de formule générale

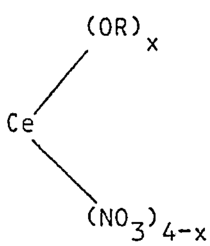

dans laquelle x est égal à 1, 2 ou 3 et H est un groupe hydrocarboné ayant de 2 à environ 20 atomes de carbone.

Selon l'invention, on fait réagir le nitrate cérique d'ammonium avec x mol d'ammoniac et un alcool répondant à la formule ROH, dans laquelle R est un groupe hydrocarboné ayant de 2 à environ 20 atomes de carbone à une température dans l'intervalle d'environ -30 à environ 200°C jusqu'à ce que l'hydrocarbyloxy-nitrate cérique et le nitrate d'ammonium soient formés.

Applications : fabrication d'alcoolates cériques.

EP 0 316 213 A2

## Description

### Hydrocarbyloxy-nitrates cériques et leur procédé de fabrication

La présente invention a pour objet des hydrocarbyloxy-nitrates cériques et leur procédé de fabrication.

Les brevets des USA n° 4 489 000 et 4 663 439 délivrés le 18 décembre 1984 et le 5 mai 1987, respectivement, aux noms de Gradeff et Schreiber, proposent un procédé pour préparer des alcoolates cériques, qui consiste à faire réagir le nitrate cérique d'ammonium avec un alcool, comprenant un alcool aliphatique inférieur, en conditions anhydres en présence d'une base anhydre à une température dans l'intervalle d'environ -30 à environ 200° C, mais de préférence de 0 à environ 150° C, jusqu'à ce qu'il se forme l'alcoolate cérique et le nitrate de la base ; les alcoolates cériques d'alcools supérieurs peuvent être préparés par transétherification de l'alcoolate cérique résultant par l'alcool supérieur, soit simultanément, soit successivement, à une température dans la gamme d'environ -30 à environ 200° C, en déplaçant ainsi l'alcool aliphatique inférieur et en formant l'alcoolate cérique de l'alcool supérieur, tout en distillant l'alcool aliphatique inférieur libre au cours de la transétherification, pour que celle-ci soit complète ; les nitrates formés pendant la réaction peuvent être séparés du mélange de réaction et les aloooolates isolés purs ou sous forme de complexes avec l'alcool, ou dans certains cas les alcoolates peuvent être utilisés en présence des nitrates sans séparation du mélange de réaction.

Le procédé est un perfectionnement considérable par rapport à la technique décrite par Bradley et autres dans J.C.S 1956 pages 2260-2264. Comme le tétrachlorure de cérium est instable, Bradley et autres ont choisi comme produit de départ le complexe hexachlorure de dipyridinium-cérium.

Le dioxyde de cérium est d'abord converti en sulfate cérique d'ammonium. L'hydroxyde cérique pur est précipité d'une solution aqueuse de sulfate cérique d'ammonium et lavé énergiquement. L'hydroxyde cérique fraîchement préparé, en suspension dans l'alcool absolu, est traité par le chlorure d'hydrogène anhydre et ensuite on ajoute de la pyridine, qui forme le complexe insoluble hexachlorure de dipyridinium-cérium $(Py)_2CeCl_6$ Le complexe est filtré, séché et utilisé pour préparer directement le méthylate, l'éthylate et l'isopropylate, tandis que les alcoolates correspondant aux groupes propyle, butyle, sec-butyle, néopentyle et n-pentyle sont préparés à partir de l'isopropylate par échange d'alcool, c'est-à-dire par transétherification. Le méthylate et l'éthylate ont également été préparés par échange à partir de l'isopropylate.

Le procédé breveté de Gradeff et autres évite la nécessité décrite par Bradley et autres de préparer d'abord l'hydroxyde cérique à partir du sel cérique, dans leur cas le sulfate cérique d'ammonium, et de convertir ensuite l'hydroxyde en le chlorure qui doit être stabilisé sous forme du complexe de pyridine. Le procédé de l'invention est direct et économique et utilise en outre un produit disponible dans le commerce qui est relativement peu coûteux, le nitrate cérique d'ammonium ou cérinitrate ou héxanitratocérate (IV) d'ammonium $Ce(NO_3)_4.2NH_4NO_3$ ou $Ce(NO_3)_6(NH_4)_2$.

Gradeff, Schreiber, Brooks et Sievers ont indiqué dans Inorganic Chemistry, 24 (1985), page 1110, que l'alooolate cérique se forme facilement et quantitativement lorsque l'on fait réagir le nitrate cérique d'ammonium (CAN) dissous dans un alcool de bas poids moléculaire tel que méthanol, éthanol ou alcool isopropylique, avec une base appropriée. Il est nécessaire que le CAN soit en solution avant l'addition de la base. Comme les alcools supérieurs solubilisent moins le CAN, ils peuvent être utilisés en mélange avec le méthanol. Dans ce cas, le méthylate est probablement formé d'abord et solubilisé sous la forme d'un complexe avec l'alcool supérieur. L'échange des alcools a lieu très facilement à des températures relativement basses. Dans certains cas, on peut ajouter au mélange de réaction un solvant tel que benzène, hexane ou toluène pour maintenir l'alcoolate en solution et faciliter la séparation du nitrate de sodium ou d'ammonium. Lorsque l'on utilise l'ammoniac anhydre comme base, il suffit de 4 mol, comme il est nécessaire pour la stoechiométrie de la réaction en conditions anhydres :

$$(NO_3)_4Ce.2NH_4NO_3 + 4ROH + 4NH_3 \rightarrow Ce(OR)_4 + 6NH_4NO_3$$

Lorsque l'on utilise un alcoolate alcalin au lieu d'ammoniac, il est préférable de prévoir un certain excès (jusqu'à 2 équivalents) par rapport aux 4 équivalents nécessaires.

$$(NO_3)_4Ce.2NH_4NO_3 + 4ROH + 6NaOCH_3 \rightarrow Ce(OR)_4 + 6NaNO_3 + 2NH_3 + 6CH_3OH$$

L'excès d'alcoolate alcalin convertit le $NH_4NO_3$ en nitrate alcalin et ammoniac, ce qui rend moins ambigu le choix du traitement et plus facile l'établissement du bilan de produits

Gradeff, Schreiber et Mauermann, décrivent dans Journal of the Less-Common, Metals, 126 (1986), pages 335-338, la préparation d'isopropylate cérique à partir de nitrate cérique d'ammonium dans le diméthoxyéthane (DME) et l'isopropanol (IPA). La préparation peut être mise en oeuvre quantitativement en une durée de l'ordre de quelques heures et un procédé est décrit à cet effet. Cependant, les auteurs remarquent que lorsque l'on utilise l'ammoniac au lieu d'isopropylate de sodium, la réaction n'est complète qu'avec difficulté, à cause d'un équilibre apparent mettant en jeu des composés ayant des liaisons cérium-nitrate de structure inconnue. Tandis que la préparation de l'isopropylate cérique en solution dans le système DME-IPA en utilisant l'isopropylate de sodium est quantitative, les tentatives pour utiliser l'ammoniac comme base n'ont pas réussi à donner un résultat net.

En l'absence de DME, la réaction avec $NH_3$ dans l'IPA est rapide et complète ; la séparation de $NH_4NO_3$ et de $Ce(OiPr)_4$ présente principalement des difficultés. En présence de DME, le problème semble se situer dans l'étape de réaction. On a d'abord penser que le DME agit par coordination sur la réactivité du CAN mais on n'en a pas trouvé de preuve décisive. Comme $Ce(OiPr)_4$ présente une solubilité considérable dans le mélange DME-IPA, la possibilité d'une réaction réversible a été examinée et démontrée de la manière suivante.

a) Lorsque l'on agite l'isopropylate cérique dans le DME avec $NH_4NO_3$, la couleur vire rapidement du jaune brillant à l'orangé avec dégagement de $NH_3$. L'addition d'ammoniac à ce mélange le fait passer à nouveau de l'orangé au jaune brillant. On agite pendant 3 h à la température ambiante dans le DME une quantité mesurée de $Ce(OiPr)_4$ et de $NH_4NO_3$ dans le rapport molaire 1:7. La distillation à température ambiante et les analyses du distillat par chromatographie gazeuse montrent qu'il y a libération de 2 équivalents d'IPA. La quantité de $NH_3$ n'est pas mesurée.

b) On fait réagir l'isopropylate de cérium avec 6 équivalents de $NH_4NO_3$. On sépare le liquide surnageant et on le sèche. Le spectre IR dans KBr (figure 1, tableau 1) démontre la présence de liaisons cérium-nitrate.

A la connaissance des auteurs, on n'a pas préparé directement d'autres alcoolates métalliques à partir de nitrates qui pourraient expliquer l'absence de renseignements sur les réactions d'échange dans les systèmes $M(OR)_n + NH_4NO_3$.

Les études cinétiques de ce type de réaction mettant en jeu $Ce(OiPr)_4$ progressent et les auteurs ont indiqué qu'elles seront étendues à d'autres alcoolates métalliques.

Selon la présente invention, on a déterminé que, lorsque l'on met en oeuvre cette réaction en présence d'ammoniac comme base, il se forme des hydrocarbyloxy nitrates cériques de formule (I) :

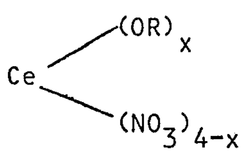

(I)

dans laquelle x est égal à 1, 2 ou 3 et R représente un groupe hydrocarboné ayant de 2 à environ 20 atomes de carbone.

Le produit de réaction peut être un seul hydrocarbyloxynitrate cérique, lorsque x est égal à 1, 2 ou 3, ou un mélange de deux quelconques ou de tous les trois, selon les proportions des réactifs. Dans le cas d'un mélange, x peut avoir une valeur fractionnaire représentant la valeur moyenne x selon les espèces présentes dans le mélange et leurs proportions relatives.

On pense que la réaction se déroule de la manière suivante :

1. $Ce(NO_3)_4.2NH_4NO_3 + xNH_3 + yROH = Ce(OR)_{4-x}(NO_3)x + 2xNH_4NO_3 + (y-x)ROH$

y est au moins égal à 2 ; x = 1, 2 ou 3.

Il est assez surprenant que la substitution partielle des groupes hydrocarbyloxy aux groupes nitrates produise un produit stable qui peut être isolé tel quel. Les alcoxy-nitrates cériques sont cependant assez stables, spécialement si on sépare tout le nitrate d'ammonium, empêchant ainsi l'inversion de la réaction ci-dessus.

Dans l'équation ci-dessus, R est un groupe hydrocarboné ayant de 2 à environ 20 atomes de carbone.

Des exemples de groupes hydrocarbonés R comprennent les groupes alkyles à chaînes droites ou ramifiées, cycloalkyles, phényle et alkylphényles, naphtyles et alkylnaphtyles.

Des exemples de groupes alkyles R comprennent les groupes éthyle, propyle, isopropyle, butyle, isobutyle, sec-butyle, tert-butyle, amyle, isoamyle, néopentyle, hexyle, isohexyle, sechexyle, heptyle, iso-heptyle, tert-heptyle, octyle, isooctyle, 2-éthyl-hexyle, tert-octyle, nonyle, isononyle, tert-nonyle, décyle, néodécyle, iso-décyle, sec-décyle, tert-décyle, dodécyle, hexadécyle, octadécyle et eicosyle.

Des exemples de groupes cycloalkyles R comprennent les groupes cyclopropyle, cyclobutyle, cyclopentyle, cyclohexyle, cycloheptyle et cyclooctyle.

Des exemples de groupes phényle et alkylphényles R comprennent les groupes phényle, phénéthyle, phénylbutyle, tolyle, xylyle, mésityle et naphtyle.

Des groupes hydrocarbonés R ayant de 1 à environ 10 atomes de carbone sont préférés.

Dans la liste des groupes R précités, ceux qui sont préférés sont choisis parmi les groupes alkyles linéaires ou ramifiés ayant de 2 à 5 atomes de carbone et le groupe cyclohexyle.

On peut utiliser un mélange de ROH, l'hydrocarbyloxynitrate cérique pouvant contenir dans ce cas des mélanges de groupes hydrocarbyloxy dans les proportions relatives des réactifs ROH.

L'hydrocarbyloxy-nitrate cérique peut être en équilibre entre les divers hydrocarbyloxy-nitrates cériques possibles, selon l'équation suivante :

2. $2(RO)_2Ce(NO_3)_2 \rightleftarrows (RO)_3Ce(NO_3) + (RO)Ce(NO_3)_3$

La proportion relative des divers hydrocarbyloxy-nitrates cériques dépend de la nature de R et du solvant utilisé.

Les hydrocarbyloxy-nitrates cériques, et en particulier les isopropoxy-nitrates cériques, sont importants pour leur utilisation pratique dans la préparation d'hydrocarbyl-oxydes cériques et en particulier d'isopropylates cériques, à partir du nitrate cérique d'ammonium. En utilisant le procédé des brevets des USA n° 4 489 000 et 4 663 439, délivrés le 18 décembre 1984 et le 5 mai 1987 respectivement, aux noms de Gradeff et Schreiber, on obtient les hydrocarbyl-oxydes et en particulier les isopropylates selon la réaction suivante :

3. $Ce(NO_3)_6(NH_4)_2 + 6NaOR \rightarrow 6NaNO_3 + 2NH_3 + Ce(OR)_4 + 2ROH$

La quantité d'hydrocarbyl-oxyde de métal alcalin anhydre ou de l'alcoolate base est la quantité stoechiométrique, puisque la fonction du cation métal alcalin de la base est de fixer le nitrate du nitrate cérique d'ammonium de départ. On peut utiliser un excès mais il n'est pas nécessaire et dans certains cas pas souhaitable car il pourrait ne pas être facile à séparer de l'hydrocarbyl-oxyde (alcoolate) cérique.

On peut voir d'après l'équation que l'on n'a pas besoin d'alcool supplémentaire. On peut cependant utiliser un excès d'alcool libre.

Comme le montre la réaction, il faut 6 mol d'hydrocarbyl-oxyde (alcoolate) de sodium. Comme c'est un réactif coûteux, ceci augmente considérablement le coût du procédé. Cependant, si le produit de départ est le dihydrocarbyl-oxyde-dinitrate cérique, l'hydrocarbyl-oxyde cérique correspondant peut se former selon l'équation suivante :

4. 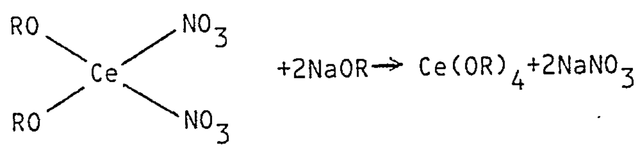


On voit d'après l'équation 4 que lorsque l'on utilise le dialcoxy-dinitrate cérique, il faut seulement 2 mol de l'alcoolate de sodium au lieu de 6 dans la réaction 3.

Donc, les alcoxy-nitrates cériques constituent une voie nouvelle et moins coûteuse d'accès aux alcoolates cériques, à partir du nitrate cérique d'ammonium, de l'ammoniac et de l'alcool, selon l'équation réactionnelle 1, suivie de la réaction avec avec NaOR selon l'équation 4.

Conformément à la présente invention, on prépare le composé de formule (I) en faisant réagir le nitrate cérique d'ammonium, de l'ammoniac et un alcool répondant à la formule ROH dans laquelle R est un groupe hydrocarboné ayant de 2 à environ 20 atomes de carbone.

On peut donc faire appel à un alcool aliphatique à chaîne droite ou ramifiée, à un alcool cycloaliphatique, à un alcool aromatique ou à un phénol.

Les alcools aliphatiques saturés sont préférés, par exemple éthanol, propanol, isopropanol, butanol, isobutanol, sec-butanol, tert-butanol, pentanol, isopentanol, sec-pentanol, néopentanol, tert-pentanol, hexanol, heptanol, isoheptanol, octanol, isooctanol, éthyl-2-hexanol, sec-octanol, tert-octanol, nonanol, isononanol, néodécanol, décanol, dodécanol, tétradécanol, octadécanol et alcool eicosylique. Cependant, on peut aussi utiliser des alcools alcényliques.

Les alcools cycloaliphatiques qui peuvent être utilisés comprennent le cyclopropanol, le cyclobutanol, le cyclopentanol, le cyclohexanol, le cycloheptanol et le cyclooctanol.

Les alcools aromatiques et phénols comprennent les suivants : alcool benzylique, phénol, naphtol, alcool naphtylique, α-phényl-éthanol, β-phényléthanol, β-phényl-butanol et alcool mésitylique. Les alcools ayant de 2 à 10 atomes de carbone sont préférés.

Les alcools aliphatiques linéaires ou ramifiés ayant de 2 à 5 atomes de carbone et le cyclohexanol constituent des matières premières de choix.

Lorsque l'alcool est liquide dans les conditions de réaction, il peut également servir de solvant et on peut donc utiliser un excès sans inconvénient, l'excès étant de préférence récupéré après la réaction complète et recyclé en vue de sa réutilisation. On peut également utiliser d'autres solvants inertes en plus du réactif alcool, par exemple benzène, hexane et acétonitrile. D'autres solvants utiles sont les éthers de glycols tels que diméthoxy-1,2-éthane, diméthoxy-1,2-propane, diméthoxy-1,3-propane et tétrahydrofuranne. Tout solvant présent peut facilement être séparé du produit de réaction par distillation sous pression atmosphérique ou sous pression réduite, après la fin de la réaction.

Il est souhaitable et préférable que la réaction se déroule en conditions anhydres, bien que l'on puisse tolérer de faibles quantités d'eau, à une température dans la gamme d'environ -30 à environ 200°C, de préférence d'environ 0 à environ 150°C, mieux encore à la température ambiante (le plus souvent comprise entre 15 et 25°C), suivant le système solvant utilisé.

L'ammoniac anhydre sert de base et c'est la seule base utilisée dans le procédé de l'invention. L'ammoniac est utilisé au moins en quantité de x mol suffisante pour séparer 2+x mol de nitrate d'ammonium du mélange

de réaction, d'aprés les proportions stoechiométriques selon l'équation 2 ci-dessus. Par cette séparation, on peut arrêter la réaction au stade de l'hydrocarbyloxy-nitrate cérique. On peut utiliser un excès, c'est-à-dire plus de 2+x mol d'ammoniac, et il peut favoriser la séparation du nitrate d'ammonium et faciliter l'isolement de l'hydrocarbyloxy-nitrate de cérium.

Par exemple, pour obtenir un dialcoxy-dinitrate de cérium, il est nécessaire de séparer 4 mol de $NH_4NO_3$. La quantité d'ammoniac nécessaire à cet effet ne peut pas s'expliquer par les seules proportions stoechiométriques. De manière plus pratique, l'équation 1 peut s'écrire comme suit :

$$Ce(NO_3)_y2NH_4NO_3 + zNH_3 + yROH \rightleftharpoons Ce(NO_3)_{4-x}(OR)_x + (2+x)NH_4NO_3 + (y-x)ROH + (z-x)NH_3$$

$x = < 4 > 0$ ; $y \geqq x$ ; z est au moins égal à x, mais de préférence supérieur.

Dans l'exemple V, z=8 Théoriquement, les 4 groupes nitro doivent tous avoir réagi et la quantité de $NH_4NO_3$ doit avoir été de 6 mol. Par contre, il ne se forme que 4,2 mol, ce qui correspond à un composé de formule $Ce(NO_3)_{4-2,8}(OR)_{2,8}$. De l'avis des inventeurs, des variations dans les conditions opératoires et les caractéristiques expérimentales entraîneraient des variations du composé. Il est évidemment surprenant que même l'utilisation d'un excès d'ammoniac produise des alcoxy-nitrates et non pas le tétraalcoolate attendu comme indiqué dans l'art antérieur.

La durée de réaction n'est pas critique. La réaction se poursuit jusqu'à ce que l'hydrocarbyloxy-nitrate désiré soit formé. Ceci peut durer de 10 min à plusieurs heures, mais il n'est pas nécessaire de mettre en oeuvre la réaction au-delà d'une durée de réaction de 5 h. Ordinairement, la réaction est complète en 1/2 h à 3 h.

La réaction peut se dérouler assez rapidement à la température ambiante et, si c'est le cas, elle aura lieu très vraisemblablement aussi à des températures bien inférieures à la température ambiante, jusqu'à -30° C, mais il n'y a ordinairement pas de raison de mettre en jeu les frais supplémentaires de refroidissement du mélange de réaction. La limite supérieure de la température de réaction est imposée par la volatilité du mélange de réaction ou de l'un de ses composants et leur température de décomposition, et par les réactions secondaires du nitrate cérique d'ammonium. A des températures élevées, au-dessus de 50° C, il est difficile de maintenir l'ammoniac dans le système. Il n'y a pas de raison d'utiliser une température supérieure au point d'ébullition du mélange de réaction à la pression atmosphérique. Compte tenu des facteurs ci-dessus, la température de réaction n'a pas besoin de dépasser 200° C.

La quantité d'alcool mis en jeu est au moins la quantité stoechiométrique y nécessaire pour réagir avec le nitrate cérique d'ammonium, mais on peut aussi utiliser des quantités supérieures. On utilisera bien entendu des quantités plus que stoechiométriques lorsque l'alcool doit également servir de solvant, suivant la dilution nécessaire du mélange de réaction.

Le mélange de réaction contient du nitrate d'ammonium et celui-ci peut être séparé de l'hydrocarbyloxy-nitrate pendant le traitement Ce sel est moins soluble dans le mélange de réaction que le produit de réaction, l'hydrocarbyloxy-nitrate, et peut être séparé par filtration et séparé ainsi du produit de réaction. Le mélange de réaction peut aussi être repris dans un solvant inerte, tel que benzène, pentane ou hexane, dans lequel l'hydrocarbyloxynitrate est soluble et le nitrate d'ammonium insoluble, le nitrate d'ammonium étant alors séparé par filtration ou centrifugation.

Selon les conditions de réaction et de traitement, l'hydrocarbyloxy-nitrate peut être isolé sous forme d'associations avec une ou plusieurs molécules d'alcool ou de solvant coordonné.

Pour certaines applications, les hydrocarbyloxy-nitrates de cérium peuvent être utilisés sous la forme où ils existent dans le mélange de réaction à la fin de la réaction, sans réellement les isoler du mélange de réaction, ou en les séparant du nitrate d'ammonium, ce qui économise des frais de traitement et de manipulation.

Par exemple, dans le cas où l'hydrocarbyloxy-nitrate cérique est utilisé comme intermédiaire dans la préparation d'un tétraalcoolate cérique différent, il n'est pas nécessaire d'isoler l'hydrocarbyloxy-nitrate cérique du mélange de réaction. Tout ce qu'il faut est que le nitrate d'ammonium soit séparé par filtration. L'alcoolate de sodium du tétraalcoolate cérique désiré est ensuite ajouté au mélange de réaction et la réaction de l'hydrocarbyloxy-nitrate cérique pour former le tétraalcoolate cérique a lieu ensuite dans les mêmes conditions que décrit ci-dessus, conduisant à la transéthérification pour former le produit désiré.

Lorsque l'hydrocarbyloxy-nitrate cérique doit être utilisé comme produit de départ pour la préparation de tétraalcoolates cériques, les conditions opératoires sont celles décrites par Gradeff et Schreiber dans les brevets des USA n° 4 489 000 et 4 663 439 (EP-A-0 134 161 et EP-A-0 183 603), substituant les hydrocarbyloxy-nitrates cériques aux nitrates cériques d'ammonium et bien entendu réduisant la quantité du réactif alcoolate de métal alcalin. En conséquence, leur description dans le brevet n° 4 489 000, col. 3, ligne 5 à col. 4, ligne 51 et dans le brevet n° 4 663 439, col. 3, ligne 35 à col. 4, ligne 60 est ainsi incorporée par référence.

Dans l'esprit des inventeurs, les exemples suivants représentent des modes de mise en oeuvre préférés de l'invention.

## Exemple I

Préparation du diisopropoxy-dinitrate cérique

On met en suspension 7 g (équivalant à 0,01277 mol) de nitrate cérique d'ammonium dans 20 ml (17 g) de diméthoxyéthane et on agite pendant environ 5 min. L'addition de 1,8 g (0,03 mol) d'isopropanol augmente la solubilité du complexe cérique avec le diméthoxyéthane. On ajoute ensuite au mélange une quantité de gaz ammoniac équivalant à 0,02554 mol, provoquant la formation immédiate d'un précipité blanc. L'addition du gaz ammoniac ne nécessite pas plus de 30 s, parce qu'à la fin de cette durée tout le nitrate cérique d'ammonium s'est dissous et l'agitation devient difficile à cause de la grande quantité de précipité de nitrate de sodium. Ensuite, on ajoute 20 ml d'éther diéthylique et on continue à agiter pendant encore 10 min. On sépare ensuite le précipité par filtration sur un verre fritté et on évapore à siccité le filtrat limpide rouge foncé, pour donner le diisopropoxy-dinitrate cérique sous forme d'une huile rouge foncé.

Exemple II

En suivant le même mode opératoire qu'à l'exemple I, on prépare le diisopropoxy-dinitrate cérique. A la solution de ce complexe contenant 61,23 mmol de cérium dans 80 ml d'alcool isopropylique et 40 ml de diméthoxyéthane, on ajoute lentement 19,62 g (122,46 mmol) de benzoate de sodium. On filtre le mélange de réac tion après repos pendant 6 d à la température ambiante, ce qui donne un précipité blanc et une solution rouge. On sèche la solution, on redissout dans le diméthoxyéthane et on ajoute du pentane pour précipiter le composé, on filtre et on sèche le composé. L'analyse par RMN d'un échantillon purifié indique la formation de $Ce(O\text{-}isoC_3H_7)_2(O_2CC_6H_5)_2$. On redissout le composé brut dans le pentane, on filtre et on place dans la neige carbonique. Il précipite 11,1 g (30,8 mmol) d'un composé jaune correspondant à un rendement de 50,23%.

Exemples III à V

On dissout 1 mol de nitrate cérique d'ammonium dans un mélange de 790 g de diméthoxyéthane et 543 g d'isopropanol (fort excès) à la température ambiante. On ajoute de l'ammoniac dans 3 proportions molaires, 2,4 et 8, provoquant la formation immédiate de nitrate d'ammonium sous forme d'un précipité blanc. Le nitrate d'ammonium est filtré, séché et pesé.

| Exemple | $NH_3$ ajouté (mol) | $NH_4NO_3$ récupéré (mol) | produit |
|---|---|---|---|
| III | 2 | 2,6 | $Ce(NO_3)_{2.7}(OisoC_3H_7)_{1.3}$ |
| IV | 4 | 4 | $Ce(NO_3)_2(OisoC_3H_7)_2$ |
| V | 8 | 4,2 | $Ce(NO_3)_{1.2}(OisoC_3H_7)_{2.8}$ |

Dans ces conditions, en utilisant un excès d'isopropanol, il faut plus que la quantité théorique de $NH_3$ (exemple IV) pour obtenir le diisopropoxy-dinitrate cérique désiré qui correspond à la formation de 4 mol de $NH_4NO_3$. Le doublement de la quantité de $NH_3$ ne déplace que légèrement vers la droite l'équilibre de la réaction, exemple V, ce qui montre que le produit désiré est celui, préféré même si x est supérieur à la valeur théorique. Ceci permet de mettre en oeuvre facilement le procédé.

On fait réagir une portion du diisopropoxy-nitrate cérique obtenu à l'exemple IV, tel qu'il est contenu dans le mélange de réaction après filtration du nitrate d'ammonium, avec 2 équivalents molaires d'isopropylate de Na. On sépare par filtration 2 mol de $NH_4NO_3$ du mélange de réaction qui contient le tétraisopropylate cérique.

**Revendications**

1. Hydrocarbyloxy-nitrates cériques, caractérisés en ce qu'ils répondent à la formule générale :

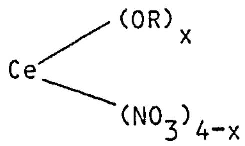

dans laquelle x est égal à 1, 2 ou 3 et R est un groupe hydrocarboné ayant de 2 à environ 20 atomes de carbone.

2. Hydrocarbyloxy-nitrates cériques selon la revendication 1, caractérisés en ce que le groupe R est un groupe alkyle à chaîne droite ou ramifiée, un groupe cycloalkyle, un groupe phényle ou alkylphényle, un groupe naphtyle ou alkylnaphtyle.

3. Hydrocarbyloxy-nitrates cériques selon la revendication 1, caractérisés en ce que le groupe R est un groupe alkyle linéaire ramifié.

4. Hydrocarbyloxy-nitrates cériques selon l'une des revendications 2 et 3, caractérisés en ce que le groupe R a de 2 à 10 atomes de carbone.

5. Hydrocarbyloxy-nitrates cériques selon l'une des revendications 1 à 5, caractérisés en ce que le groupe R est un groupe alkyle linéaire ou ramifié ayant de 2 à 5 atomes de carbone ou un groupe cyclohexyle.

6. Procédé de fabrication des hydrocarbyloxy-nitrates cériques décrits dans l'une des revendications 1 à 5, caractérisé par le fait qu'il consiste à faire réagir le nitrate cérique d'ammonium, avec au moins x moles d'ammoniac et un alcool répondant à la formule ROH, dans laquelle R est un groupe hydrocarboné ayant de 2 à environ 20 atomes de carbone, à une température dans l'intervalle d'environ -30° à environ 200° C jusqu'à ce que l'hydrocarbyloxy-nitrate cérique et le nitrate d'ammonium soient formés.

7. Procédé selon la revendication 6, caractérisé en ce que l'on utilise un excès d'ammoniac d'au moins 2+x mol.

8. Procédé selon la revendication 6, caractérisé en ce que l'alcool est présent en quantité d'au moins x mol.

9. Procédé selon la revendication 6, caractérisé en ce que le groupe R est un groupe alkyle linéaire ou ramifié.

10. Procédé selon l'une des revendications 6 et 9, caractérisé en ce que le groupe R a de 2 à 10 atomes de carbone.

11. Procédé selon l'une des revendications 9 et 10, caractérisé en ce que le groupe R est un groupe alkyle linéaire ou ramifié ayant de 2 à 5 atomes de carbone ou un groupe cyclohexyle.

12. Procédé selon la revendication 6, caractérisé en ce qu'il est mis en oeuvre en solution dans un solvant inerte.

13. Procédé selon la revendication 12, caractérisé en ce que le solvant est l'alcool dont on veut former l'hydrocarbyloxynitrate.

14. Procédé selon la revendication 6, caractérisé en ce qu'il est mis en oeuvre à une température de réaction dans la gamme de la température ambiante à environ 50° C.

15. Procédé selon la revendication 6, caractérisé en ce que l'hydrocarbyl-oxyde de cérium produit est récupéré et séparé du nitrate d'ammonium par filtration.

16. Procédé pour fabriquer des alcoolates cériques, caractérisé en ce que l'on fait réagir le nitrate cérique d'ammonium et un alcool avec x mol d'ammoniac à une température dans l'intervalle d'environ -30° C à environ 200° C jusqu'à ce que l'alcoxy-nitrate cérique soit formé, on sépare le nitrate d'ammonium et ensuite on fait réagir l'alcoxy-nitrate cérique avec un alcoolate de métal alcalin de l'alcool de l'alcoolate cérique désiré à une température dans la gamme d'environ -30°C à environ 50°C jusqu'à ce que la réaction se produise pour former l'alcoolate cérique et le nitrate du métal alcalin.

17. Procédé selon la revendication 16, caractérisé en ce que l'alcool présent dans le mélange de réaction est l'alcool correspondant à l'alcoolate de métal alcalin.

18. Procédé selon la revendication 16, caractérisé en ce que l'alcool est un alcool autre que celui correspondant à l'alcoolate de métal alcalin.

19. Utilisation des hydrocarbyloxy-nitrates cériques à la fabrication des alcoolates cériques.